(19) Europäisches Patentamt
European Patent Office
Office européen des brevets

(11) Veröffentlichungsnummer: **0 045 996**
**B1**

# (12) EUROPÄISCHE PATENTSCHRIFT

(45) Veröffentlichungstag der Patentschrift: **27.03.85**

(51) Int. Cl.⁴: **C 07 D 229/00**, C 08 G 18/80, C 08 G 18/79

(21) Anmeldenummer: **81200892.8**

(22) Anmeldetag: **10.08.81**

(54) **Verfahren zur Herstellung von uretdiongruppenhaltigen Polyadditionsprodukten sowie die danach hergestellten Produkte.**

(30) Priorität: **13.08.80 DE 3030572**

(43) Veröffentlichungstag der Anmeldung: **17.02.82 Patentblatt 82/7**

(45) Bekanntmachung des Hinweises auf die Patenterteilung: **27.03.85 Patentblatt 85/13**

(84) Benannte Vertragsstaaten: **BE CH DE FR GB IT LI LU NL SE**

(56) Entgegenhaltungen:
**FR - A - 2 221 481**
**FR - A - 2 268 840**
**FR - A - 2 298 566**

**Die Akte enthält technische Angaben, die nach dem Eingang der Anmeldung eingereicht wurden und die nicht in dieser Patentschrift enthalten sind.**

(73) Patentinhaber: **CHEMISCHE WERKE HÜLS AG, Postfach 1320, D-4370 Marl 1 (DE)**

(72) Erfinder: **Disteldorf, Josef, Dr., Am Sengenhoff 2a, D-4690 Herne 1 (DE)**
Erfinder: **Gras, Rainer, Dr., An der Ziegelei 91, D-4690 Herne 2 (DE)**
Erfinder: **Hübel, Werner, Dr., Birnenbruchstrasse 34, D-4690 Herne 1 (DE)**
Erfinder: **Wolf, Elmar, Dr., Stauffenbergstrasse 7, D-4350 Recklinghausen (DE)**
Erfinder: **Schnurbusch, Horst, Dr., Overwegstrasse 36, D-4690 Herne 1 (DE)**

(74) Vertreter: **Stell, Hanna, Dipl.-Chem., RSP PATENTE - PB 15 Postfach 1320, D-4370 Marl 1 (DE)**

EP 0 045 996 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Uretdiongruppen aufweisende Polyadditionsprodukte sind bekannt und werden z. B. in der DE-AS 2 420 475 beschrieben. Solche Verbindungen werden durch Reaktion von einem Uretdion aus z. B. Toluylendiisocyanat oder Hexamethylendiisocyanat und Diolen oder Diaminen erhalten. Zur Herstellung der in der DE-A-2 420 475 beanspruchten Uretdiongruppen aufweisenden Polyadditionsverbindungen soll auch das Uretdion des Isophorondiisocyanats geeignet sein; es finden sich allerdings weder Beispiele noch näher Erläuterungen.

Das Fehlen der Beispiele und dergleichen ist nicht überraschend, da die Voraussetzung für die Herstellung der genannten Uretdiongruppen aufweisenden Polyadditionsverbindungen darin besteht, daß das zur Kettenverlängerung mit Diolen eingesetzte Uretdion-Diisocyanat eine NCO-Funktionalität von 2 besitzt. Bei einer NCO-Funktionalität des Uretdion-Diisocyanat von >2 muß bei dessen Umsetzung mit z. B. Diolen bereits mit mindestens teilweiser Gelierung gerechnet werden.

Mit den zum Stand der Technik zählenden Dimerisierungskatalysatoren (tert. Phosphine) war es nämlich bis jetzt gar nicht möglich, ein isocyanuatfreies Uretdion des Isophorondiisocyanats (IPDI) herzustellen. Ein nach der DE-A-1 670 720 bzw. DE-A-1 934 763 hergestelltes Uretdion-Isophorondiisocyanat enthält je nach Reaktionsbedingungen noch ca. 20—40 Gew.-% des trimeren IPDI (Isocyanurat-Isophorondiisocyanat) im Gemisch.

Für eine gezielte Weiterreaktion mit z. B. Diolen zur Herstellung von wertvollen Ausgangsverbindungen für die Polyurethanchemie kommt ein solches isocyanurathaltiges Uretdion-Isophorondiisocyanat nicht in Frage.

Diese gezielte Weiterreaktion wurde erst durch Einsatz eines reinen isocyanuratfreien Uretdions aus Isophorondiisocyanat möglich. Die Herstellung dieses in reiner Form und nicht als Gemisch mit Isocyanurat vorliegenden Uretdions, das in der Hitze zu über 98% in Isophorondiisocyanat rückspaltbar ist, ist nicht Gegenstand dieser Patentanmeldung. Sie erfolgt dadurch, daß ,man Isophorondiisocyanat gegebenenfalls in einem inerten organischen Lösungsmittel mit Hilfe eines Katalysators der allgemeinen Formel

$$X_mP(NR_2)_{3-m}$$

wobei

m = 0, 1, 2
X = Cl, OR, R
R = gleiche oder verschiedene Alkyl-, Aralkyl-, gegebenenfalls alkylsubstituierte Cycloalkylreste und 2(R-1 H) Bestandteile eines gemeinsamen Ringes bedeuten

bei Temperaturen von 0—80° C vorzugsweise 10—30° C, dimerisiert, und das gebildete 1,3-Diazacyclobutandion-2,4 nach einem Umsatz von 5—70, vorzugsweise 20—50%, ohne vorgehende Desaktivierung des Katalysators aus dem Reaktionsgemisch durch Dünnschichtdestillation als Rückstand sowie Katalysator und Monomeres als Destillat isoliert.

Das so hergestellte Uretdion des IPDI ist bei Raumtemperatur hochviskos ($>10^6$m Pas; bei 60° C $13 \cdot 10^3$m Pas; bei 80° C $1{,}4 \cdot 10^3$m Pas). Sein NCO-Gehalt liegt im Bereich von 16,8—18%; d. h. daß mehr oder minder hohe Anteile an Polyuretdionen des IPDI im Reaktionsprodukt vorliegen müssen. Der Monomergehalt liegt bei <1%. Der NCO-Gehalt des Reaktionsproduktes nach dem Erhitzen auf 180—200° C beträgt 37,1—37,7% NCO.

Bei der Umsetzung des Uretdions mit den Diolen soll das NCO/OH-Verhältnis von Uretdion-Isophorondiisocyanat und Diol 1 : 0,5—1 : 0,9 bzw. 0,5 : 1—0,9 : 1 betragen. Le nach diesem Verhältnis enthalten diese Uretdionpolyadditionsprodukte endständig freie NCO-Gruppen oder — für besondere Fälle — endständige OH-Gruppen.

Für bestimmte Anwendungen hat sich als zweckmäßig erwiesen, bei den Uretdion-Isophorondiisocyanat/Diol-Polyadditionsaddukten die endständigen NCO-Gruppen ganz oder wenigstens teilweise mit Monoalkoholen, primären oder sekundären Monoaminen umzusetzen.

Gegenstand der Erfindung ist daher ein Verfahren zur Herstellung von uretdiongruppenhaltigen Polyadditionsprodukten aus uretdiongruppenhaltigen Isophorondiisocyanat und Diolen, dadurch gekennzeichnet, daß man ein uretdiongruppenhaltiges Isophorondiisocyanat, das in der Hitze zu 98% in IPDI rückspalbar ist, mit Diolen in einem NCO/OH-Verhältnis von 1 : 0,5—1 : 0,9 bzw. 0,5 : 1—0,9 : 1 umsetzt, und das so erhaltene Additionsprodukt gegebenenfalls ganz oder teilweise mit Monoalkoholen bzw. Monoaminen umsetzt.

Weiterer Gegenstand der Erfindung sind die wie beansprucht hergestellten uretdiongruppenhaltigen Polyadditionsprodukte aus uretdionhaltigem Isophorondiisocyanat, das in der Hitze zu 98% in IPDI rückspaltbar ist, und Diolen und gegebenenfalls Monoalkoholen bzw. Monoaminen.

Bei der Umsetzung von Uretdion-Isophorondiisocyanat mit dem Diol kann man dabei so verfahren, daß das Diol in einem Guß oder eben durch allmähliches Eintragen dem Uretdion-Isophorondiisocyanat zugeführt wird.

Beispiele derartiger zweiwertiger Alkohole sind Ethylenglykol, Propylen-(1,2) und -(1,3)glykol, 2-Ethylhexandiol-(1,3), Hexandiol-(1,6), Oktandiol-(1,8), Neopentylglykol, 1,4-Bishydroxymethylcyclohexan, 3(4), 8(9)-Bishydroxymethyltricyclodecan, 2-Methylpropandiol-(1,3), 3-Methylpentandion-(1,5), ferner Diethylenglykol, Triethylenglykol, Tetraethylenglykol, Dipropylenglykol und Dibutylenglykol. Bevorzugt findet Butylenglykol-(1,4) als verknüpfendes Diol zum Aufbau der erfindungsgemäßen Polyuretdion-Polyurethane Anwendung.

Bei der Umsetzung werden die Reaktionspartner in den angegebenen Mengenverhältnissen gemischt. Im allgemeinen wird, wie bereits angeführt, die Isocyanatkomponente vorgelegt und das Diol zugegeben. Die Reaktion kann in Substanz oder auch in Gegenwart geeigneter Lösungsmittel durchgeführt werden. Geeignete Lösungsmittel sind z. B. Benzol, Toluol, Methyl- bzw Ethylglykolacetat, Dimethylformamid, Xylol, aliphatische Kohlenwasserstoffe, Ketone, wie Aceton, Methylbutylketon, Methylisobutylketon, Cyclohexanon und chlorierte aromatische Kohlenwasserstoffe sowie beliebige Gemische dieser und anderer inerter Lösungsmittel.

Die Umsetzung findet im allgemeinen bei Temperaturen von 50—120°C vorzugsweise 60—90°C statt. Die Reaktionskomponenten werden so lange bei den angegebenen Temperaturen erhitzt, bis alle OH-Gruppen unter Bildung von Urethangruppen umgesetzt sind. Dies dauert je nach Reaktionstemperatur 0,5—5 h. Es können auch die Isocyanatpolyaddition beschleunigenden Katalysatoren mitverwendet werden; bevorzugt finden organische Zinnverbindungen, wie Zinn-II-acetat, Zinn-II-octoat, Zinn-IIlaurat, Dibutyl-zinndiacetat, Dibutylzinndilaurat, Dibutyl-zinnmaleat oder Dioctylzinndiacetat als Katalysatoren Verwendung. Die Katalysatoren werden im allgemeinen in einer Menge zwischen 0,01 und 0,5 Gew.-% bezogen auf die Gesamtmenge der eingesetzten Reaktanten, eingesetzt.

Die Aufarbeitung der Reaktionsansätze erfolgt in der Regel so, daß man die Polyuretdion-Polyurethane vom gegebenenfalls mitverwendeten Lösungsmittel befreit. Das kann durch einfaches Entfernen des Lösungsmittels im Vakuum erfolgen. Besonders geeignet zur Beseitigung des Lösungsmittels ist die Schmelzextrusion in einer Ausdampfschnecke.

Besonders vorteilhaft sind weiterhin Reaktionsprodukte, die durch Umsetzung der beschriebenen Uretdion-Isophorondiisocyanat/Diol-Additionsverbindungen mit Monoalkoholen erhalten werden. Die Reaktion der Additionsverbindungen mit den Monoalkoholen wird so durchgeführt, daß alle oder wenigstens ein Teil der NCO-Gruppen mit Monoalkoholen umgesetzt werden. Man geht so vor, daß man das Uretdion-Isophorondiisocyanat mit dem Diol unter den bereits beschriebenen Bedingungen zur Reaktion bringt und nach erfolgter Umsetzung nicht abkühlt, sondern unter Beibehaltung der Temperatur dem Reaktionsprodukt den Monoalkohol zugibt. Die Reaktionsmischung wird dann so lange weiter erhitzt, bis pro eingesetztes OH die äquivalente Menge NCO umgesetzt worden ist. Die Isolierung der Reaktionsprodukte erfolgt ähnlich wie vorstehend angeführt.

Geeignete einwertige Alkohole sind Methanol, Ethanol, n-Butanol, 2-Ethylhexanol, n-Decanol, Cyclohexanol. Die Uretdion-Polyadditionsprodukte mit Monoalkoholen eignen sich im besonderen Maße als Bindemittelkomponente für PUR-Pulverlacke, die beim Härten kein Blockierungsmittel abspalten.

Anstelle der Monoalkohole können auch primäre oder sekundäre Monoamine eingesetzt werden. Als Monoamine eignen sich z. B. n-Propylamin, n-Butylamin, n-Hexylamin, Dibutylamin, Dicyclohexylamin.

Bei der Umsetzung der Uretdion-Polyadditionsprodukte mit primären oder sekundären Monoaminen empfiehlt es sich, das Amin portionsweise zuzugeben, da die $NH_2$/NCO-Reaktion sehr schnell und mit großer Wärmetönung abläuft. Die Isolierung der Reaktionsprodukte erfolgt wie vorher beschrieben.

Weitere vorteilhafte Polyadditionsverbindungen aus Uretdion-Isophorondiisocyanat und Diolen sind solche mit endständigen OH-Gruppen, d. h. daß das Uretdion mit dem Diol in einem NCO/OH-Verhältnis von 0,5 : 1—0,9 : 1 umgesetzt wird.

Bei den Polyadditionsprodukten, nämlich

1. Addukten mit endständigen freien NCO-Gruppen;
2. solche deren NCO-Gruppen ganz oder teilweise mit monoalkoholen oder Monoaminen umgesetzt sind;
3. solche mit endständigen OH—Gruppen

handelt es sich im allgemeinen um Verbindungen des Molekulargewichtsbereichs 900—5 000, vorzugsweise 1 000—3 000. Die Polyadditionsprodukte weisen einen Schmelzpunkt von 80—140°C, vorzugsweise 100—130°C auf. Sie eignen sich insbesondere als Härter für Zerewittinoff-aktive Wasserstoffatome aufweisende höherfunktionelle (thermoplastische) Verbindungen. In Kombination mit derartigen Zerewittinoff-aktive Wasserstoffatomen aufweisenden Verbindungen bilden die Polyadditionsprodukte oberhalb 140°C, vorzugsweise 160—180°C zu hochwertigen Kunststoffen aushärtbare Systeme. Das bedeutendste Anwendungsgebiet für derartige Systeme ist ihre Verwendung als Bindemittelkomponente für PUR-Pulverlacke und Einkomponenten-Einbrennlacke.

Die erfindungsgemäßen Verbindungen sollen als Zwischenprodukt zur Herstellung von Kunststoffen, Lacken und Schaumstoffen Verwendung finden. Sie sind deshalb besonders wertvoll, weil sie wegen ihres niedrigen Dampfdruckes physiologisch ungefährlich sind. Besonders geeignet sind sie zur Herstellung von lösungsmittelhaltigen und lösungsmittelarmen Ein- und Zweikomponenten-PUR-Lacken, wie coil-coiting- oder highsolid-Lacken, ferner PUR-Pulverlacken sowie lösungsmittelfreien Ein- und Zweikomponentenbeschichtungen.

Beispiel A

Herstellung des uretdiongruppenhaltigen Isophorondiisocyanats

100 Gew.-Teile Isophorondiisocyanat wurden mit 1 Gew.-Teil Tris-(dimethyl-amino)-phosphin versetzt und 20 h bei Raumtemperatur stehen gelassen. Der NCO-Gehalt dieses Gemisches nach dieser Zeit betrug 31 Gew.-%, d. h., daß ca. 40% des eingesetzten Isophorondiisocyanats reagiert hatten. Anschließend wurde dieses Gemisch der Dünnschichtdestillation bei 130° C und 0,133 mbar unterworfen. Der Rückstand war katalysatorfrei und hatte einen NCO-Gehalt von 17,6%. Wurde der Rückstand 30—60 Minuten bei 180° C erhitzt, so stieg der NCO-Gehalt auf 37,1—37,7%. Dieser »sogenannte Heißwert« war ein direktes Maß für den Gehalt an Uretdiongruppen im Reaktionsprodukt.

Beispiel B

Herstellung der Isophorondiisocyanat-Uretdion-Addukte

— Allgemeine Herstellungsvorschrift —

Das gemäß A 1 hergestellte Isophorondiisocyanat-Uretdiondiisocyanat wird vorgelegt, gegebenenfalls im Lösungsmittel wie Aceton, Methylenchlorid, Toluol oder Xylol und auf 50°—100° C erwärmt. Unter starkem Rühren und Inertgasatmosphäre fügt man dem Uretdiondiisocyanat das Diol so zu, daß die Reaktionstemperatur 110° C nicht übersteigt. Die Umsetzung, die durch titrimetrische NCO-Bestimmung kontrolliert wird, ist nach 2—5 h bei ca. 100° C beendet. Bei der teilweisen oder vollständigen Blockierung der freien NCO-Gruppen wird die erforderliche Menge Alkohol zugefügt, und bei 110° C die Reaktion zu Ende geführt. Der Reaktionsablauf wird ebenfalls mittels titrimetrischer NCO-Bestimmung kontrolliert. Nach Abkühlen, gegebenenfalls Absaugen und Trocknen und gegebenenfalls Zerkleinern der Reaktionsprodukte erhält man schwach gelbliche, freifließende Pulver. Die IR-Spektren der Vernetzungsprodukte weisen bei 1 760—1 780 $cm^{-1}$ die charakteristischen, intensiven Banden der Uretdiongruppen auf; je nach Blockierungsgrad sind keine oder nur schwach ausgeprägte NCO-Banden IR-spektroskopisch nachweisbar.

Uretdion-Addukte

| Ausgangskomponenten | | | | Reaktionsprodukte | | | |
|---|---|---|---|---|---|---|---|
| Beispiel | IPDI-Uretdion Mol | Diol Mol | Mono-alkohol Mol | Gesamt-NCO-% | Freie-NCO-% | Schmelzpunkt °C | DTA °C |
| 1 | 2 | 1 B | | 25,11 | 7,6 | 81—86 | 41—55 |
| 2 | 3 | 2 B | | 22,14 | 4,6 | 93—96 | 45—57 |
| 3 | 4 | 3 B | | 20,5 | 3,3 | 98—102 | 49—60 |
| 4 | 5 | 4 B | | 19,4 | 2,5 | 100—106 | 47—61 |
| 5 | 4 | 5 B | | 11,8 | <0,1 | 97—105 | 50—60 |
| 6 | 3 | 2 HD | | 21,1 | 4,4 | 76—81 | 45—55 |
| 7 | 4,5 | 3,5 HD | | 19,1 | 2,8 | 77—82 | 44—53 |
| 8 | 5 | 4 HD | | 18,9 | 2,4 | 80—85 | 47—58 |
| 9 | 3 | 2 MP | | 20,9 | 4,6 | 75—78 | 44—55 |
| 10 | 5 | 4 MP | | 18,6 | 2,7 | 80—84 | 47—57 |

(Fortsetzung)

| Ausgangskomponenten | | | | Reaktionsprodukte | | | |
|---|---|---|---|---|---|---|---|
| Beispiel | IPDI-Uretdion Mol | Diol Mol | Mono-alkohol Mol | Gesamt-NCO-% | Freie-NCO-% | Schmelz-punkt °C | DTA °C |
| 11 | 4,5 | 3,5 NPG | | 19,3 | 2,7 | 81—86 | 48—57 |
| 12 | 4,5 | 3,5 DEG | | 19,2 | 2,8 | 78—80 | 42—51 |
| 13 | 4,5 | 3,5 EG | | 20,5 | 2,9 | 81—85 | 43—52 |
| 14 | 4 | 3 DMC | | 18,8 | 3,0 | 86—91 | 49—59 |
| 15 | 4,5 | 3,5 TMH | | 18,0 | 2,5 | 75—81 | 42—51 |
| 16 | 3 | 2 B | 1 EH | 16,68 | 1,05 | 104—108 | 55—65 |
| 17 | 3 | 2 B | 2 EH | 15,21 | 0,48 | 95—100 | 53—58 |
| 18 | 4 | 3 B | 1 EH | 17,81 | 1,62 | 110—115 | 56—61 |
| 19 | 4,5 | 3,5 B | 1 EH | 17,88 | 1,2 | 107—118 | 62—69 |
| 20 | 4,5 | 3,5 B | 2 EH | 16,2 | 0,2 | 104—110 | 60—70 |
| 21 | 4,5 | 3,5 B | 2 M | 16,8 | 0,4 | 108—117 | 58—67 |
| 22 | 4,5 | 3,5 B | 2 E | 16,7 | 0,3 | 106—111 | 59—68 |
| 23 | 4,5 | 3,5 EG | 2 EH | 16,4 | 0,35 | 108—119 | 61—71 |
| 24 | 4,5 | 3,5 HD | 2 EH | 15,8 | 0,51 | 102—107 | 58—63 |
| 25 | 4,5 | 3,5 MP | 2 EH | 15,7 | 0,41 | 101—109 | 57—66 |
| 26 | 4,5 | 3,5 B | 1 M | 18,1 | 1,3 | 110—115 | 62—78 |
| 27 | 4,5 | 3,5 B | 2 DBA | 15,3 | 0 | 117—123 | 65—77 |

B Butandiol-1,4
DBA Dibutylamin
DEG Diethylenglykol
DMC 1,4-Bis-(hydroxymethyl)cyclohexan
E Ethanol
EG Ethylenglykol
EH 2-Ethylhexanol
HD Hexandiol-1,6
M Methanol
MP 3-Methylpentandiol-1,5
NPG Neopentylglykol
TMH 2,2,4(2,4,4)-Trimethylhexandiol-1,6

## Patentansprüche

1.Verfahren zur Herstellung von uretdiongruppenhaltigen Polyadditionsprodukten aus uretdiongruppenhaltigem Isophorondiisocyanat und Diolen, dadurch gekennzeichnet, daß man ein uretdiongruppenhaltiges Isophorondiisocyanat, das in der Hitze zu 98% in Isophorondiisocyanat rückspaltbar ist, mit Diolen in einem NCO/OH-Verhältnis von 1 : 0,5—1 : 0,9 bzw. 0,5 : 1—0,9 : 1 umsetzt, und das so erhaltene Additionsprodukt gegebenenfalls ganz oder teilweise mit Monoalkoholen bzw. Monoaminen umsetzt.

2. Verfahren nach Anspruch 1, dadurch gekennzeichnet, daß als Diole Ethylenglykol, Butandiol-(1,4), Hexandiol-(1,6), Diethylenglykol, 3-Methylpentadiol-(1,5) eingesetzt werden.

3. Verfahren nach Anspruch 1, dadurch gekennzeichnet, daß als Monoalkohole Methanol, Ethanol, 2-Ethylhexanol, Butanol, Cyclohexanol eingesetzt werden.

4. Verfahren nach Anspruch 1, dadurch gekennzeichnet, daß die Reaktionskomponenten in Lösung umgesetzt werden, und nach erfolgter Umsetzung das Lösungsmittel entfernt wird.

5. Uretdiongruppenhaltige Polyadditionsprodukte aus uretdionhaltigem Isophorondiisocyanat, das in der Hitze zu 98% in Isophorondiisocyanat rückspaltbar ist, und Diolen und gegebenenfalls Monoalkohole bzw. Monoamine, hergestellt nach Ansprüchen 1—4.

## Claims

1. Process for the preparation of polyaddition products, containing uretdione groups, from isophorone diisocyanate containing uretdione groups and from diols, characterised in that an isophorone diisocyanate containing uretdione groups, which upon heating can be redecomposed to the extent of 98% into isophorone diisocyanate, is reacted with diols in an NCO/OH ratio of 1 : 0.5—1 : 0.9 or 0.9 : 1—0.9 : 1 and the addition products thus obtained is optionally reacted entirely or partially with monoalcohols or monoamines respectively.

2. Process according to Claim 1, characterised in that ethylene glycol, butane-1,4-diol, hexane-1,6-diol, diethylene glycol or 3-methylpentane-1,5-diol are employed as diols.

3. Process according to Claim 1, characterised in that methanol, ethanol, 2-ethylhexanol, butanol and cyclohexanol are employed as monoalcohols.

4. Process according to Claim 1, characterised in that the reactants are reacted in solution and that after the reaction has taken place, the solvent is removed.

5. Polyaddition products, containing uretdione groups, of isophorone diisocyanate which upon heating can be redecomposed to the extent of 98% into isophorone diisocyanate, and diols, and, optionally, monoalcohols or monoamines, the products having been prepared according to Claims 1—4.

## Revendications

1. Procédé pour la fabrication de produits de polyaddition contenant des groupes uretdione, à partir de diisocyanate d'isophoron et de diols, caractérisé en ce que l'on fait réagir un diisocyanate d'isophoron contenant des groupes uretdione, qui peut rétrograder à la chaleur jusqu'à 98% en diisocyanate d'isophoron, avec des diols, dans un rapport NCO/OH de 1 : 0,5 à 1 : 0,9 ou 0,5 : 1 à 0,9 : 1, et fait réagir le produit d'addition ainsi obtenu éventuellement totalement ou partiellement avec des monoalcools ou des monoamines.

2. Procédé suivant la revendication 1, caractérisé en ce que l'on met en oeuvre, comme diols, les éthylèneglycol, butane-diol-(1,4), hexanediol-(1,6), diéthylèneglycol, 3-méthylpentanediol-(1,5).

3. Procédé suivant la revendication 1, caractérisé en ce que l'on utilise, comme monoalcools, les méthanol, éthanol, 2-éthylhexanol, butanol, cyclohexanol.

4. Procédé suivant la revendication 1, caractérisé en ce que l'on fait réagir les composants de la réaction en solution, et que la réaction effectuée, on élimine le solvant.

5. Produit de polyaddition contenant des groupes uretdione à partir de diisocyanate d'isophoron contenant des groupes uretdione, qui peut rétrograder à la chaleur jusqu'à 98% en diisocyanate d'isophoron, et de diols, et éventuellement de monoalcools ou de monoamines, fabriqué suivant les revendications 1 à 4.